# EUROPÄISCHE PATENTSCHRIFT

(11) **EP 1 670 741 B1**
(45) Veröffentlichungstag und Bekanntmachung des Hinweises auf die Patenterteilung: **03.03.2010**
(21) Anmeldenummer: 04765590.7
(22) Anmeldetag: 24.09.2004
(51) Int. Cl.: C07C 51/265, C07C 51/31, B01J 23/22, B01J 27/198, B01J 37/02

(54) **VERFAHREN ZUR HERSTELLUNG EINES GASPHASENOXIDATIONSKATALYSATORS MIT DEFINIERTER VANADIUMOXID-TEILCHENGRÖSSENVERTEILUNG**
PROCESS FOR THE PREPARATION OF A GAS PHASE OXIDATION CATALYST WITH DEFINED VANADIUM OXIDE PARTICLE SIZE DISTRIBUTION
PROCEDE POUR LA PREPARATION D'UN CATALYSEUR D'OXYDATION EN PHASE GAZEUSE A REPARTITION GRANULOMETRIQUE D'OXYDE DE VANADIUM DEFINIE

(30) Priorität: 26.09.2003 DE 10344846
(43) Veröffentlichungstag der Anmeldung: 21.06.2006
(73) Patentinhaber: BASF SE, 67056 Ludwigshafen (DE)
(72) Erfinder: STORCK, Sebastian, 68167 Mannheim (DE); ZÜHLKE, Jürgen, 67346 Speyer (DE); NETO, Samuel, 01099 Dresden (DE); ROSOWSKI, Frank, 68165 Mannheim (DE); RUMMEL, Wolfgang, 50931 Köln (DE)
(74) Vertreter: Reitstötter - Kinzebach
(86) Internationale Anmeldenummer: PCT/EP2004/010749
(87) Internationale Veröffentlichungsnummer: WO 2005/030692

(56) Entgegenhaltungen:
- EP-A- 1 181 097
- US-A- 5 792 719
- US-B1- 6 586 361

## Beschreibung

Die Erfindung betrifft ein Verfahren zur Herstellung eines Titandioxid und Vanadiumoxid umfassenden Gasphasenoxidationskatalysators mit definierter Vanadiumoxid-Teilchengrößenverteilung.

Eine Vielzahl von Carbonsäuren und/oder Carbonsäureanhydriden wird technisch durch die katalytische Gasphasenoxidation von aromatischen Kohlenwasserstoffen, wie Benzol, den Xylolen, Naphthalin, Toluol oder Durol, in Festbettreaktoren hergestellt. Man kann auf diese Weise z. B. Benzoesäure, Maleinsäureanhydrid, Phthalsäureanhydrid, Isophthalsäure, Terephthalsäure oder Pyromellithsäureanhydrid erhalten. Im Allgemeinen leitet man ein Gemisch aus einem sauerstoffhaltigen Gas und dem zu oxidierenden Ausgangsmaterial durch Rohre, in denen sich eine Schüttung eines Katalysators befindet. Zur Temperaturregelung sind die Rohre von einem Wärmeträgermedium, beispielsweise einer Salzschmelze, umgeben.

Als Katalysatoren haben sich für diese Oxidationsreaktionen so genannte Schalenkatalysatoren bewährt, bei denen die katalytisch aktive Masse schalenförmig auf einem inerten Trägermaterial, wie Steatit aufgebracht ist. Als katalytisch aktiver Bestandteil der katalytisch aktiven Masse dieser Schalenkatalysatoren dient im allgemeinen neben Titandioxid Vanadiumpentoxid. Des weiteren können in der katalytisch aktiven Masse in geringen Mengen eine Vielzahl anderer oxidischer Verbindungen enthalten sein, die als Promotoren die Aktivität und Selektivität des Katalysators beeinflussen.

Zur Herstellung derartiger Schalenkatalysatoren wird eine wässrige Suspension der Aktivmassenbestandteile und/oder deren Vorläuferverbindungen auf das Trägermaterial bei erhöhter Temperatur aufgesprüht, bis der gewünschte Aktivmassenanteil am Katalysatorgesamtgewicht erreicht ist.

Die DE-A 2550686 beschreibt ein Verfahren, bei dem eine wässrige Lösung, die Titantetrachlorid und ein Vanadium(IV)-salz enthält, auf einen Träger aufgebracht wird.

Bei der in der DE-A 1442590 veranschaulichten Herstellung wird zu einer Lösung von Vanadyloxalat, Formamid und Wasser feinteiliges Titandioxid in der Anatas-Modifikation gegeben. Die erhaltene Aufschlämmung wird auf inerte Katalysatorträger aufgebracht.

Die WO 00/12214 und die US 6,586,361 beschreiben ein Herstellungsverfahren, bei dem ein Gemisch aus Titandioxid, Vanadyloxalat, organischem Binder und gegebenenfalls Promotoren durch Aufsprühen in der Dragiertrommel, Beschichtung in einer Wirbelschicht oder Pulverbeschichtung schalenförmig in zwei konzentrischen Schichten auf inerte Trägerringe aufgebracht wird.

Weitere Katalysatoren, die V₂O₅ und TiO₂ auf einem inerten Träger enthalten, gehen aus der US 5,792,719 und der WO 01/03832 hervor.

Die EP-A 539878 veranschaulicht die Herstellung von Katalysatoren zur Herstellung von Phthalsäureanhydrid in der Gasphase. Ammoniummetavanadat wird in wässriger Oxalsäurelösung gelöst und gemeinsam mit Promotoren gerührt. Anschließend erfolgt die Zugabe von TiO₂, das aus Titansulfat nach dem Sulfat-Verfahren hergestellt ist. Die erhaltene Suspension wird homogenisiert und bei erhöhter Temperatur auf Katalysatorträger aufgesprüht.

Gemäß der DE-A 2106796 und der DE-A 19633757 wird eine wässrige Suspension von Anatas und Titandioxid-Hydrat, V₂O₅ und einer organischen Binderkomponente auf die Träger aufgebracht.

Die bekannten Herstellungsverfahren lassen sich hinsichtlich der verwendeten Vanadiumquelle in zwei Klassen unterteilen: In einem Fall wird als Vanadiumquelle eine lösliche Vanadium(IV)-Verbindung, wie Vanadyloxalat, verwendet. Die Reduktion zum Vanadium(IV) erfolgt mit organischen Reduktionsmitteln, wie Oxalsäure. Im anderen Fall werden unlösliche Vanadium(V)-verbindungen wie V₂O₅ zur wässrigen Suspension gegeben. Da hier das Erfordernis des Reduktionsmittels entfällt, sind die Kosten für die Einsatzstoffe geringer. Nachteilig ist allerdings, dass die V₂O₅-Teilchen beim Beschichtungsvorgang in einer Wirbelschicht zur Entmischung neigen und nicht vollständig auf den zu beschichtenden Träger gelangen, sondern teilweise z. B. mit der Prozessluft entweichen oder sich als Belag an der Beschichtungsapparatur absetzen. Um den Verlust auszugleichen, muss ein Überschuss an V₂O₅ verwendet werden. Es ist wünschenswert, den erforderlichen Überschuss so gering wie möglich zu halten.

Der Erfindung liegt die Aufgabe zu Grunde, ein wirtschaftliches Verfahrung zur Herstellung von Titandioxid und Vanadiumoxid enthaltenden Gasphasenoxidationskatalysatoren bereitzustellen.

Es wurde nun gefunden, dass die Effizienz der Beschichtung stark von der Teilchengrößenverteilung des in der Beschichtungssuspension suspendierten V₂O₅ abhängt.

Die Erfindung betrifft ein Verfahren zur Herstellung eines Katalysators für Gasphasenoxidationen, bei dem man auf einen fluidisierten inerten Träger eine Suspension von TiO₂- und V₂O₅-Teilchen aufbringt, worin wenigstens 90 Vol.-% der V₂O₅-Teilchen einen Durchmesser von 20 µm oder weniger und wenigstens 95 Vol.-% der V₂O₅-Teilchen einen Durchmesser von 30 µm oder weniger aufweisen.

Vorzugsweise weisen wenigstens 90 Vol.-% der V₂O₅-Teilchen einen Durchmesser von 15 µm oder weniger und wenigstens 95 Vol.-% der V₂O₅-Teilchen einen Durchmesser von 20 µm oder weniger auf.

In besonders bevorzugten Ausführungsformen weisen wenigstens 60 Vol.-% der V₂O₅-Teilchen einen Durchmesser von 4 µm oder weniger auf, wenigstens 80 Vol.% der V₂O₅-Teilchen einen Durchmesser von 10 µm oder weniger, wenigstens 90 Vol.-% der V₂O₅-Teilchen einen Durchmesser von 15 µm oder weniger und wenigstens 95 Vol.-% der V₂O₅-Teilchen einen Durchmesser von 20 µm oder weniger auf.

Vorzugsweise weisen wenigstens 50 Vol.-% der V₂O₅-Teilchen einen Durchmesser von mehr als 2 µm auf. Der volumenbezogene D₅₀-Wert liegt vorzugsweise im Bereich von 2,0 bis 2,5 µm.

Die volumenbezogene Teilchengrößenverteilung wird für die Zwecke der vorliegenden Anmeldung geeigneterweise mittels Laserbeugung und Auswertung nach der Fraunhofer-Methode bestimmt. Bei dieser Methode wird parallel ausgerichtetes Laserlicht an den Teilchen gebeugt. Jedes Teilchen erzeugt ein für seine Größe charakteristisches Beugungsbild. Das Beugungsspektrum wird mit Detektoren erfasst und mit einem Mikrocomputer die Korngrößenverteilung als Volumenverteilung berechnet.

Vanadiumoxide geeigneter Teilchengrößenverteilung lassen sich durch ausreichend lange Mahlung in geeigneten Mühlen herstellen. Es eignen sich z. B. Prallmühlen, Wälzmühlen, Schwingmühlen, Mahlkörpermühlen oder Sturzmühlen. Mahlkörpermühlen sind bevorzugt. Sie bestehen aus einem horizontal gelagerten zylindrischen Arbeitsraum, der um einen festen Drehpunkt rotiert. Der Arbeitsraum ist mit Mahlkörpern in der Regel unterschiedlicher Größe gefüllt. Das Mahlgut befindet sich im Lückenvolumen der Mahlkörper. Als Mahlkörper werden verschleißfeste geschmiedete oder gegossene Stahlkugeln, Stangen bzw. Stangenabschnitte verwendet. In Abhängigkeit von der Drehzahl der Mühle werden sich bestimmte Bewegungsformen der Mahlkörper und damit unterschiedliche Beanspruchungsarten des Mahlgutes wie Reibung, Schlag, Prall und Druck einstellen, wodurch die größeren Teilchen des Mahlguts zerteilt werden.

Vorzugsweise enthält die katalytisch aktive Masse im calzinierten Zustand, bezogen auf die Gesamtmenge der katalytisch aktiven Masse, 1 bis 40 Gew.-% Vanadiumoxid, berechnet als V₂O₅, und 60 bis 99 Gew.-% Titandoxid, berechnet als TiO₂. Die katalytisch aktive Masse kann daneben bis zu 1 Gew.-% einer Cäsiumverbindung, berechnet als Cs, bis zu 1 Gew.-% einer Phosphorverbindung, berechnet als P und bis zu 10 Gew.-% Antimonoxid, berechnet als Sb₂O₃ enthalten.

Neben den fakultativen an Zusätzen Cäsium und Phosphor können im Prinzip in der katalytisch aktiven Masse in geringen Mengen eine Vielzahl anderer oxidischer Verbindungen enthalten sein, die als Promotoren die Aktivität und Selektivität des Katalysators beeinflussen, beispielsweise indem sie seine Aktivität absenken oder erhöhen. Als solche Promotoren seien beispielhaft die Alkalimetalloxide, insbesondere außer dem genannten Cäsiumoxid, Lithium-, Kalium- und Rubidiumoxid, Thallium(I)oxid, Aluminiumoxid, Zirkoniumoxid, Eisenoxid, Nickeloxid, Kobaltoxid, Manganoxid, Zinnoxid, Silberoxid, Kupferoxid, Chromoxid, Molybdänoxid, Wolframoxid, Iridiumoxid, Tantaloxid, Nioboxid, Arsenoxid, Antimonoxid, Ceroxid genannt. In der Regel wird aus dieser Gruppe Cäsium als Promotor verwendet.

Ferner kommen von den genannten Promotoren noch bevorzugt als Zusätze die Oxide von Niob und Wolfram in Mengen von 0,01 bis 0,50 Gew.-%, bezogen auf die katalytisch wirksame Masse in Betracht. Als die Aktivität erhöhenden aber die Selektivität vermindernden Zusatz kommen vor allem oxidische Phosphorverbindungen insbesondere Phosphorpentoxid in Betracht.

Das eingesetzte Titandioxid besteht vorteilhaft aus einem Gemisch eines TiO₂ mit einer BET-Oberfläche von 5 bis 15 m²/g und eines TiO₂ mit einer BET-Oberfläche von 15 bis 50 m²/g. Man kann auch ein einzelnes Titandioxid mit einer BET-Oberfläche von 5 bis 50 m/g, vorzugsweise 13 bis 28 m/g verwenden.

Als inertes Trägermaterial können praktisch alle Trägermaterialien des Standes der Technik, wie sie vorteilhaft bei der Herstellung von Schalenkatalysatoren für die Oxidation aromatischer Kohlenwasserstoffe zu Aldehyden, Carbonsäuren und/oder Carbonsäureanhydriden eingesetzt werden, Verwendung finden, beispielsweise Quarz (SiO₂), Porzellan, Magnesiumoxid, Zinndioxid, Siliciumcarbid, Rutil, Tonerde (Al₂O₃), Aluminiumsilikat, Steatit (Magnesiumsilikat), Zirkoniumsilikat, Cersilikat oder Mischungen dieser Trägermaterialien. Das Trägermaterial ist in der Regel nicht-porös. Der Ausdruck "nicht-porös" ist dabei im Sinne von "bis auf technisch unwirksame Mengen an Poren nicht-porös" zu verstehen, da technisch unvermeidlich eine geringe Anzahl Poren im Trägermaterial, das idealerweise keine Poren enthalten sollte, vorhanden sein können. Als vorteilhafte Trägermaterialien sind insbesondere Steatit und Siliciumcarbid hervorzuheben. Die Form des Trägermaterials ist für die erfindungsgemäßen Präkatalysatoren und Schalenkatalysatoren im Allgemeinen nicht kritisch. Beispielsweise können Katalysatorträger in Form von Kugeln, Ringen, Tabletten, Spiralen, Röhren, Extrudaten oder Splitt verwendet werden. Die Dimensionen dieser Katalysatorträger entsprechen denen üblicherweise zur Herstellung von Schalenkatalysatoren für die Gasphasenpartialoxidation von aromatischen Kohlenwasserstoffen verwendeten Katalysatorträgern. Bevorzugt wird Steatit in Form von Kugeln mit einem Durchmesser von 3 bis 6 mm oder von Ringen mit einem äußeren Durchmesser von 5 bis 9 mm und einer Länge von 3 bis 8 mm und einer Wandstärke von 1 bis 2 mm verwendet.

Beim erfindungsgemäßen Verfahren erfolgt das Aufbringen der Schicht(en) des Schalenkatalysators durch Aufsprühen einer Suspension von TiO₂ und V₂O₅, die gegebenenfalls Quellen der oben genannten Promotorelemente enthält, auf den fluidisierten Träger. Vor der Beschichtung wird die Suspension vorzugsweise ausreichend lange, z. B. 2 bis 30 Stunden, insbesondere 12 bis 25 Stunden, gerührt, um Agglomerate der suspendierten Feststoffe aufzubrechen und eine homogene Suspension zu erhalten. Die Suspension hat typischerweise einen Feststoffgehalt von 20 bis 50 Gew.-%. Das Suspensionsmedium ist im Allgemeinen wässrig, z. B. Wasser selbst oder ein wässriges Gemisch mit einem wassermischbaren organischen Lösungsmittel, wie Methanol, Ethanol, Isopropanol, Formamid und dergleichen.

In der Regel werden der Suspension organische Binder, bevorzugt Copolymere, vorteilhaft in Form einer wässrigen Dispersion, von Vinylacetat/Vinyllaurat, Vinylacetat/Acrylat, Styrol/Acrylat sowie Vinylacetat/Ethylen zugesetzt. Die Binder sind als wässrige Dispersionen handelsüblich, mit einem Feststoffgehalt von z. B. 35 bis 65 Gew.-%. Die eingesetzte Menge solcher Binderdispersionen beträgt im Allgemeinen 2 bis 45 Gew.-%, vorzugsweise 5 bis 35 Gew.-%, besonders bevorzugt 7 bis 20 Gew.-%, bezogen auf das Gewicht der Suspension.

Der Träger wird in einer Wirbelschicht- bzw. Fließbettapparatur in einem aufsteigenden Gasstrom, insbesondere Luft, fluidisiert. Die Apparate bestehen meist aus einem konischen oder kugelförmigen Behälter, bei dem das fluidisierende Gas von unten oder von oben über ein Tauchrohr eingeführt wird. Die Suspension wird über Düsen von oben, seitlich oder von unten in die Wirbelschicht eingesprüht. Vorteilhaft ist der Einsatz eines mittig bzw. konzentrisch um das Tauchrohr angeordneten Steigrohrs. Innerhalb des Steigrohres herrscht eine höhere Gasgeschwindigkeit, die die Trägerpartikel nach oben transportiert. Im äußeren Ring liegt die Gasgeschwindigkeit nur wenig oberhalb der Lockerungsgeschwindigkeit. So werden die Partikel kreisförmig vertikal bewegt. Eine geeignete Fließbettvorrichtung ist z. B. in der DE-A 4006935 beschrieben.

Bei der Beschichtung des Katalysatorträgers mit der katalytisch aktiven Masse werden im Allgemeinen Beschichtungstemperaturen von 20 bis 500 °C angewandt, wobei die Beschichtung unter Atmosphärendruck oder unter reduziertem Druck erfolgen kann. Im Allgemeinen erfolgt die Beschichtung bei 0 °C bis 200 °C, vorzugsweise bei 20 bis 150 °C, insbesondere bei 60 bis 120 °C durchgeführt.

Die katalytisch aktive Masse kann auch in zwei oder mehreren Schichten aufgebracht sein, wobei z. B. die innere Schicht oder die innere Schichten einen Antimonoxidgehalt von bis zu 15 Gew.-% und die äußere Schicht einen um 50 bis 100% verringerten Antimonoxidgehalt aufweisen. Dabei ist in der Regel die innere Schicht des Katalysators phosphorhaltig und die äußere Schicht phosphorarm oder phosphorfrei.

Die Schichtdicke der katalytisch aktiven Masse beträgt in der Regel 0,02 bis 0,2 mm, vorzugsweise 0,05 bis 0,15 mm. Der Aktivmasseanteil am Katalysator beträgt üblicherweise 5 bis 25 Gew.-%, meist 7 bis 15 Gew.-%.

Durch thermische Behandlung des so erhaltenen Präkatalysators bei Temperaturen über 200 bis 500 °C entweicht das Bindemittel durch thermische Zersetzung und/oder Verbrennung aus der aufgetragenen Schicht. Vorzugsweise erfolgt die thermische Behandlung in situ im Gasphasenoxidationsreaktor.

Die erfindungsgemäß hergestellten Katalysatoren eignen sich generell zur Gasphasenoxidation aromatischer C₆- bis C₁₀-Kohlenwasserstoffe, wie Benzol, den Xylolen, Toluol, Naphthalin oder Durol (1,2,4,5-Tetramethylbenzol) zu Carbonsäuren und/oder Carbonsäureanhydriden wie Maleinsäureanhydrid, Phthalsäureanhydrid, Benzoesäure und/oder Pyromellithsäuredianhydrid.

Zu diesem Zweck werden die erfindungsgemäß hergestellten Katalysatoren in von außen auf die Reaktionstemperatur, beispielsweise mittels Salzschmelzen, thermostatisierte Reaktionsrohre gefüllt und über die so bereitete Katalysatorschüttung das Reaktionsgas Temperaturen von im allgemeinen 300 bis 450 °C, vorzugsweise von 320 bis 420 °C und besonders bevorzugt von 340 bis 400 °C und bei einem Überdruck von im allgemeinen 0,1 bis 2,5 bar, vorzugsweise von 0,3 bis 1,5 bar mit einer Raumgeschwindigkeit von im allgemeinen 750 bis 5000 h⁻¹ geleitet.

Das dem Katalysator zugeführte Reaktionsgas wird im allgemeinen durch Vermischen von einem molekularen Sauerstoff enthaltenden Gas, das außer Sauerstoff noch geeignete Reaktionsmoderatoren und/oder Verdünnungsmittel, wie Dampf, Kohlendioxid und/oder Stickstoff, enthalten kann, mit dem zu oxidierenden, aromatischen Kohlenwasserstoff erzeugt, wobei das molekularen Sauerstoff enthaltende Gas im allgemeinen 1 bis 100 mol-%, vorzugsweise 2 bis 50 mol-% und besonders bevorzugt 10 bis 30 mol-% Sauerstoff, 0 bis 30 mol-%, vorzugsweise 0 bis 10 mol-% Wasserdampf sowie 0 bis 50 mol-%, vorzugsweise 0 bis 1 mol-% Kohlendioxid, Rest Stickstoff, enthalten kann. Zur Erzeugung des Reaktionsgases wird das molekularen Sauerstoff enthaltende Gas im allgemeinen mit 30 g bis 150 g je Nm³ Gas des zu oxidierenden, aromatischen Kohlenwasserstoffs beschickt.

Es hat sich als besonders vorteilhaft erwiesen, wenn in der Katalysatorschüttung Katalysatoren eingesetzt werden, die sich in ihrer katalytischen Aktivität und/oder chemischen Zusammensetzung ihrer Aktivmasse unterscheiden. Vorzugsweise wird bei Anwendung zweier Reaktionszonen in der ersten, also zum Gaseintritt des Reaktionsgases hin gelegenen Reaktionszone, ein Katalysator eingesetzt, der in Vergleich zum Katalysator, welcher sich in der zweiten, also zum Gasaustritt hin gelegenen Reaktionszone, befindet, eine etwas geringere katalytische Aktivität hat. Im allgemeinen wird die Umsetzung durch die Temperatureinstellung so gesteuert, dass in der ersten Zone der größte Teil der im Reaktionsgas enthaltenen aromatischen Kohlenwasserstoff bei maximaler Ausbeute umgesetzt wird. Bevorzugt werden drei- bis fünflagige Katalysatorsysteme verwendet, insbesondere drei- und vierlagige Katalysatorsysteme.

Die Erfindung wird durch die folgenden Beispiele näher veranschaulicht.

Die Messung der Teilchengrößenverteilung erfolgte mit Hilfe eines Frisch Particle Sizer "analysette 22" im Messbereich von 0,3 bis 300 µm mit einer Auflösung von 62 Kanälen. Die V₂O₅-Probe wurde zur Messung in Wasser suspendiert und in der Messzelle umgepumpt. Die Messdauer betrug 2 Scans. Die Auswertung erfolgte nach der Fraunhofer-Methode.

### Beispiel 1

54,227 kg Anatas (BET-Oberfläche 9 m²/g), 126,517 kg Anatas (BET-Oberfläche 20 m²/g), 14,195 kg V₂O₅, 3,549 kg Sb₂O₃, 0,805 kg Cäsiumcarbonat wurden in 519,035 kg entionisiertem Wasser suspendiert und gerührt, um eine homogene Verteilung zu erzielen. Das V₂O₅ hatte folgende volumenbezogene Teilchengrößenverteilung: 10 % ≤ 0,58 µm; 20 % ≤ 0,87 µm; 30 % ≤ 1,20 µm; 40 % ≤ 1,61 µm; 50 % ≤ 2,21 µm; 60 % ≤ 3,26 µm; 70 % ≤ 5,52 µm; 80 % ≤ 9,46 µm; 90 % ≤ 14,92 µm; 95 % ≤ 19,51 µm; 99,9 % ≤ 169,33 µm. Der Suspension wurden 80 kg eines organischen Binders, bestehend aus einem Copolymer aus Vinylacetet und Vinyllaurat in Form einer 50 Gew.-%igen Dispersion zugesetzt. In einer Wirbelbettbeschichtungsapparatur wurden 60 kg dieser Suspension auf 150 kg Steatitringe (Magnesiumsilikat) der Abmessungen 7 x 7 x 4 mm (Außendurchmesser x Höhe x Innendurchmesser) aufgesprüht und getrocknet. Die Beschichtung erfolgte bei Temperaturen von 80-120 °C und einer Luftmenge von 6000 m³/h.

Die Analyse der bei 400 °C kalzinierten Katalysatoren ergab einen V₂O₅-Anteil in der Aktivmasse von 6,85 Gew.-%. Der rechnerische Sollwert des V₂O₅-Anteils der geglühten Aktivmasse beträgt dagegen 7,12 Gew.-%. Es lag eine Fehlmenge von 0,27 % (absolut) vor. Um den V₂O₅-Verlust bei der Beschichtung zu kompensieren und Katalysatoren mit der vorgegebenen V₂O₅-Mengen herzustellen, musste die V₂O₅-Menge in der Suspension um 0,543 kg erhöht werden.

### Vergleichsbeispiel 2

Beispiel 1 wurde wiederholt, wobei jedoch das verwendete V₂O₅ folgende volumenbezogene Teilchengrößenverteilung aufwies: 10 % ≤ 0,62 µm; 20 % ≤ 0,93 µm; 30 % ≤ 1,25 µm; 40 % ≤ 1,63 µm; 50 % ≤ 2,10 µm; 60 % ≤ 2,76 µm; 70 % ≤ 3,84 µm; 80 % ≤ 6,27 µm; 90 % ≤ 24,24 µm; 95 % ≤ 46,58 µm; 99,9 % ≤ 300 µm.

Die Analyse der bei 400 °C kalzinierten Katalysatoren ergab einen V₂O₅-Anteil in der Aktivmasse von 5,55 Gew.-%. Gegenüber dem Sollwert von 7,12 Gew:-% lag eine Fehlmenge von 1,57 % (absolut) vor. Um den V₂O₅-Verlust bei der Beschichtung zu kompensieren und Katalysatoren mit der vorgegebenen V₂O₅-Mengen herzustellen, musste die V₂O₅-Menge in der Suspension um 3,134 kg erhöht werden.

Die vorstehenden Beispiele zeigen, dass durch Einsatz von V₂O₅ mit definierter Teilchengrößenverteilung die erforderliche Einsatzmenge verringert werden kann.

## Patentansprüche

1. Verfahren zur Herstellung eines Katalysators für Gasphasenoxidationen, bei dem man auf einen fluidisierten inerten Träger eine Suspension von TiO₂- und V₂O₅-Teilchen aufbringt, worin wenigstens 90 Vol.-% der V₂O₅-Teilchen einen Durchmesser von 20 µm oder weniger und wenigstens 95 Vol.-% der V₂O₅-Teilchen einen Durchmesser von 30 µm oder weniger aufweisen.

2. Verfahren nach Anspruch 1, wobei wenigstens 90 Vol.-% der V₂O₅-Teilchen einen Durchmesser von 15 µm oder weniger und wenigstens 95 Vol.-% der V₂O₅-Teilchen einen Durchmesser von 20 µm oder weniger aufweisen.

3. Verfahren nach Anspruch 1 oder 2, wobei wenigstens 50 Vol.-% der V₂O₅-Teilchen einen Durchmesser von mehr als 2 µm aufweisen.

4. Verfahren nach einem der Ansprüche 1 bis 3, wobei die Suspension außerdem wenigstens eine Cäsium-, Phosphor- und/oder Antimonquelle enthält.

5. Verfahren nach einem der vorhergehenden Ansprüche, wobei die aufgebrachte katalytisch aktive Masse 1 bis 40 Gew.-% Vanadiumoxid, berechnet als V₂O₅, und 60 bis 99 Gew.-% Titandioxid, berechnet als TiO₂, enthält.

6. Verfahren nach Anspruch 5, wobei die katalytisch aktive Masse, bezogen auf die Gesamtmenge der katalytisch aktiven Masse, bis zu 1 Gew.-% einer Cäsiumverbindung, berechnet als Cs, bis zu 1 Gew.-% einer Phosphorverbindung, berechnet als P und bis zu 10 Gew.-% Antimonoxid, berechnet als Sb₂O₃ enthält.

## Claims

1. A process for producing a catalyst for gas-phase oxidations, in which a suspension of TiO₂ and V₂O₅ particles is applied to a fluidized inert support, wherein at least 90% by volume of the V₂O₅ particles have a diameter of 20 µm or less and at least 95% by volume of the V₂O₅ particles have a diameter of 30 µm or less.

2. The process according to claim 1, wherein at least 90% by volume of the V₂O₅ particles have a diameter of 15 µm or less and at least 95% by volume of the V₂O₅ particles have a diameter of 20 µm or less.

3. The process according to claim 1 or 2, wherein at least 50% by volume of the V₂O₅ particles have a diameter of more than 2 µm.

4. The process according to any of claims 1 to 3, wherein the suspension further comprises at least one cesium, phosphorus and/or antimony source.

5. The process according to any of the preceding claims, wherein the catalytically active composition applied comprises from 1 to 40% by weight of vanadium oxide, calculated as V₂O₅, and from 60 to 99% by weight of titanium dioxide, calculated as TiO₂.

6. The process according to claim 5, wherein the catalytically active composition further comprises, based on the total amount of catalytically active composition, up to 1 % by weight of a cesium compound, calculated as Cs, up to 1 % by weight of a phosphorus compound, calculated as P, and up to 10% by weight of antimony oxide, calculated as Sb₂O₃.

## Revendications

1. Procédé pour la préparation d'un catalyseur pour des oxydations en phase gazeuse, dans lequel on applique une suspension de particules de TiO₂ et de V₂O₅ sur un support inerte fluidisé, au moins 90% en volume des particules de V₂O₅ présentant un diamètre de 20 µm ou moins et au moins 95% en volume des particules de V₂O₅ présentant un diamètre de 30 µm ou moins.

2. Procédé selon la revendication 1, au moins 90% en volume des particules de V₂O₅ présentant un diamètre de 15 µm ou moins et au moins 95% en volume des particules de V₂O₅ présentant un diamètre de 20 µm ou moins.

3. Procédé selon la revendication 1 ou 2, au moins 50% en volume des particules de V₂O₅ présentant un diamètre supérieur à 2 µm.

4. Procédé selon l'une quelconque des revendications 1 à 3, où la suspension contient en outre au moins une source de césium, de phosphore et/ou d'antimoine.

5. Procédé selon l'une quelconque des revendications précédentes, la masse catalytiquement active appliquée contenant 1 à 40% en poids d'oxyde de vanadium, calculé sous forme de V₂O₅, et 60 à 99% en poids de dioxyde de titane, calculé sous forme de TiO₂.

6. Procédé selon la revendication 5, la masse catalytiquement active, par rapport à la quantité totale de la masse catalytiquement active, contenant jusqu'à 1% en poids d'un composé du césium, calculé sous forme de Cs, jusqu'à 1% en poids d'un composé du phosphore, calculé sous forme de P et jusqu'à 10% en poids d'oxyde d'antimoine, calculé sous forme de Sb₂O₃.
